# EUROPÄISCHE PATENTSCHRIFT

(11) **EP 0 917 451 B1**
(45) Veröffentlichungstag und Bekanntmachung des Hinweises auf die Patenterteilung: **24.07.2002**
(21) Anmeldenummer: 98929332.9
(22) Anmeldetag: 18.05.1998
(51) Int. Cl.: A61B 19/00, G02B 7/00, F16M 11/04

(54) **SCHWENKTRÄGER, INSBESONDERE FÜR EIN OPERATIONSMIKROSKOP**
SWIVEL ARM, ESPECIALLY FOR AN OPERATION MICROSCOPE
SUPPORT PERMETTANT DE PIVOTER NOTAMMENT UN MICROSCOPE D'OPERATION

(30) Priorität: 20.05.1997 CH 116297
(43) Veröffentlichungstag der Anmeldung: 26.05.1999
(73) Patentinhaber: Leica Mikroskopie Systeme AG, 9435 Heerbrugg (CH)
(72) Erfinder: SCHÜPBACH, Rolf, CH-9444 Diepoldsau (CH)
(86) Internationale Anmeldenummer: EP9802909
(87) Internationale Veröffentlichungsnummer: WO9852484

(56) Entgegenhaltungen:
- EP-A- 0 293 228
- EP-A- 0 628 290

## Beschreibung

In der Chirurgie finden mehr und mehr Operationsmikroskope Anwendung, die infolge ihres hohen Eigengewichtes von Stativen getragen werden müssen. Eine Reihe namhafter Hersteller brachte Stative auf den Markt, die in mechanischer und statischer Hinsicht den Anforderungen der Lastaufnahme des Operationsmikroskops gut entsprechen. Die Anmelderin vertreibt z.B. Stative mit der Bezeichnung OH, die von Mitaka hergestellt wurden. Ein Beispiel für ein solches Stativ findet sich in der EP-A-628290.

Eine Weiterentwicklung dieses Stativs ist in der WO-A-97/13997 der Anmelderin beschrieben.

In der CH-A-3467/95 und in der am gleichen Tag wie die vorliegende Anmeldung eingereichten Patentanmeldung (u.Z.RAP-3779-CH) sind Weiterentwicklungen hinsichtlich der Materialverwendung und Schwingungsdämpfung dargestellt.

Die meisten der moderneren Stative verfügen über Parallelogrammträger, um die Last der Operationsmikroskope über möglichst grosse Distanzen biege- und verwindungsfrei tragen zu können, so dass die Bewegungsfreiheit und der Aktionsradius der Mikroskope möglichst gross sind.

In der EP-A-628290 ist beispielsweise ein solcher Aufbau dargestellt, der auch für den Schwenkträger, das ist jener Bauteil, an dem das Operationsmikroskop unmittelbar schwenkbar befestigt ist, Parallelogrammträger vorsieht. Parallelogrammträger haben dabei die Aufgabe, Haltekräfte für die Last, d.h. für das Operationsmikroskop, so in das Stativ einzuleiten, dass das Mikroskop beim Arretieren von Bremsen am Stativ in seiner Lage stabil verbleibt.

Als Aufgabe liegt der vorliegenden Erfindung die Weiterentwicklung dieses bekannten Schwenkträgers zugrunde. Vor allem soll auf Parallelogrammträger verzichtet werden und trotzdem eine verwindungsfreie Lagerung eines Operationsmikroskops möglich sein. Der Aktionsradius darf nicht eingeschränkt sein und der Aufbau soll stabil und anwenderfreundlich sowie möglichst spielfrei sein.

Gelöst wird diese Aufgabe durch die im kennzeichnenden Teil des Patentanspruchs 1 angegebenen Merkmale.

Der neuartige Winkelträger ist gemäss einer Weiterbildung der Erfindung mit einer Parallelschlittenführung ausgerüstet, die die Winkelposition der Winkel im Raum aufrecht hält.

Im Unterschied zum Bisherigen ersetzt sich dadurch auch der Bedarf an zwei einstellbaren Schlittenführungen für die beiden oberen parallelen Trägern einer herkömmlichen Parallellenkeranordnung. Eine einzige einstellbare Schlittenführung genügt für die Horizontalverschiebung des Lastangriffspunktes.

Die Erfindung kann bevorzugt mit einem Stativ gemäss der WO-A-97/13997 zum Einsatz gelangen; sie ist darauf jedoch nicht eingeschränkt.

Die Figuren werden zusammenhängend beschrieben. Die Figurenbeschreibung und die Bezugszeichenliste bilden eine Einheit, die sich durch die übrigen Teile der Beschreibung und Ansprüche im Sinne einer vollständigen Offenbarung gegenseitig ergänzen. Gleiche Bezugszeichen bedeuten gleiche Bauteile. Gleiche Bezugszeichen mit unterschiedlichen Indizes bedeuten ähnliche, funktionsgleiche Bauteile. Die Figuren sind nur beispielhaft und nicht zwingend proportional richtig dargestellt.

Es zeigen dabei:

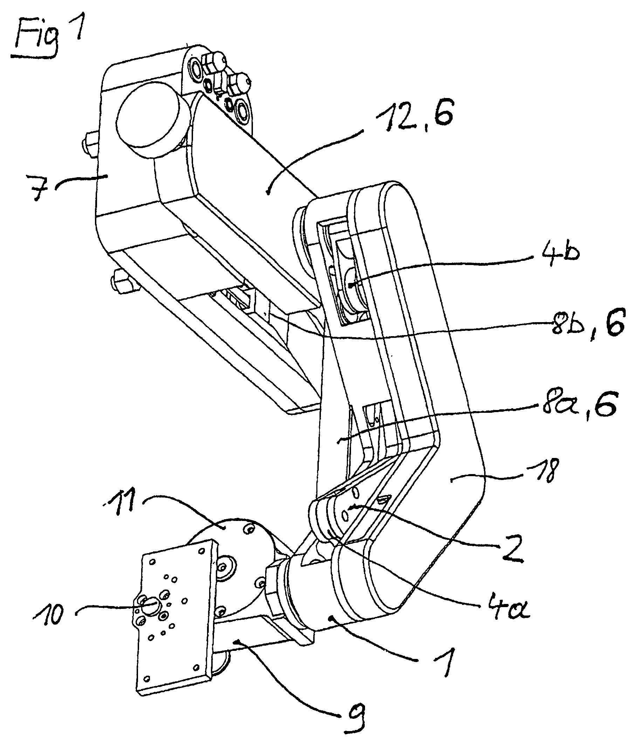

Fig.1 eine Schrägansicht eines erfindungsgemässen Schwenkträgers für ein Stativ;
Fig.2 zeigt denselben Schwenkträger in Explosionsdarstellung;
Fig.3 eine Schlittenführung für horizontale y-y-Justage des Schwenkträgers;
Fig.4 die beiden Winkelträger im Detail;
Fig.5 die montierten Winkelträger ohne Abdeckung;

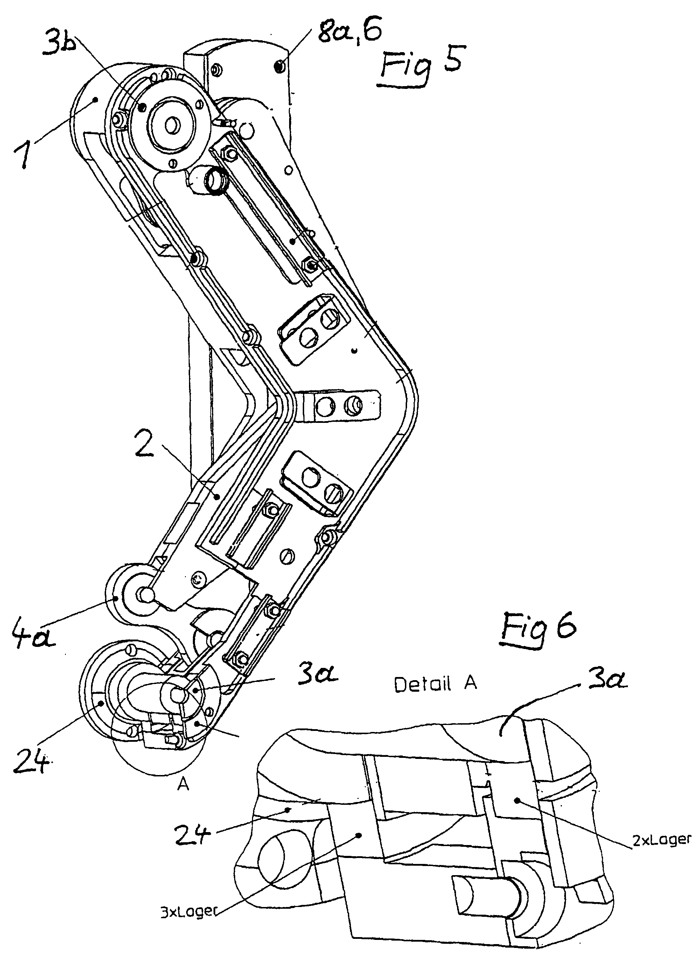

Fig.6 einen Ausschnitt der Darstellung gemäss Fig.5;

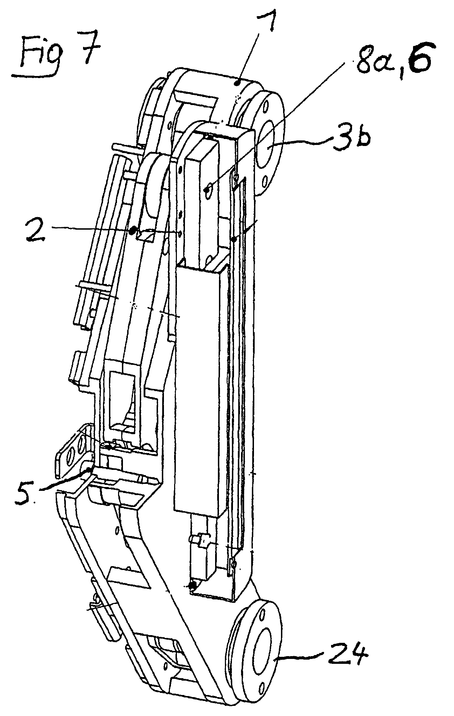

Fig.7 eine andere Ansicht auf den Zusammenbau gem. Fig.5
Fig.8 einen teilweisen Schnitt durch die Schwenkachsenaufhängung und
Fig.9 den Neigeachsenaufbau

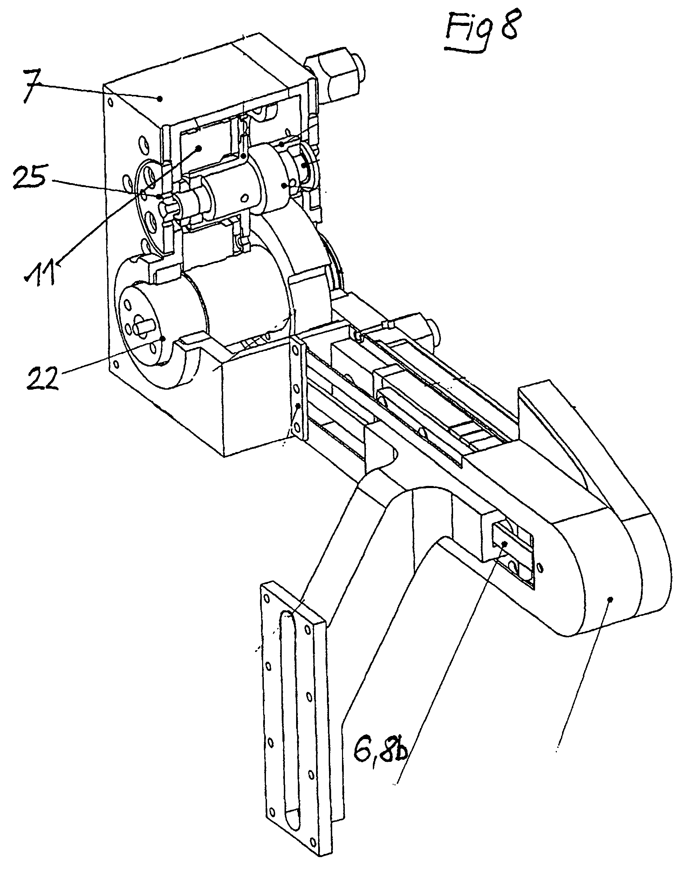

Eine Aufhängung 7 verfügt über ein Stativinterface 25 (Fig.8) mit dem sie mit dem Stativ verbunden bzw. verbindbar ist. Der Aufbau des Stativs ist dabei sekundär, wenngleich ein Aufbau gemäss der WO-A bevorzugt ist.

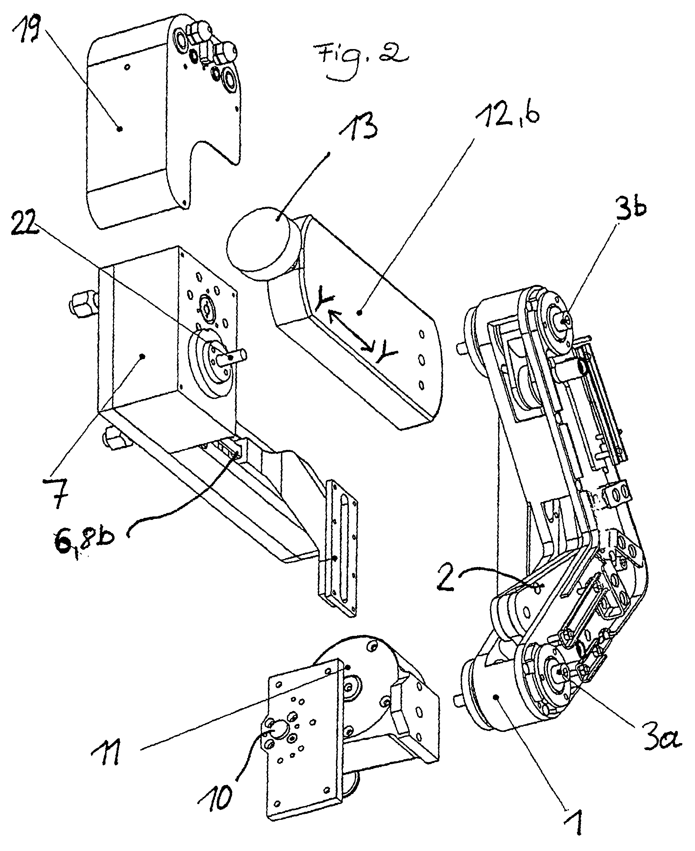

Eine bremsbare Achse 22 (Fig.2) stellt die Verbindung zu einer Aufnahme 21 her, die in einer Schlittenführung 12 in x-x Richtung verschieblich einstellbar ist.

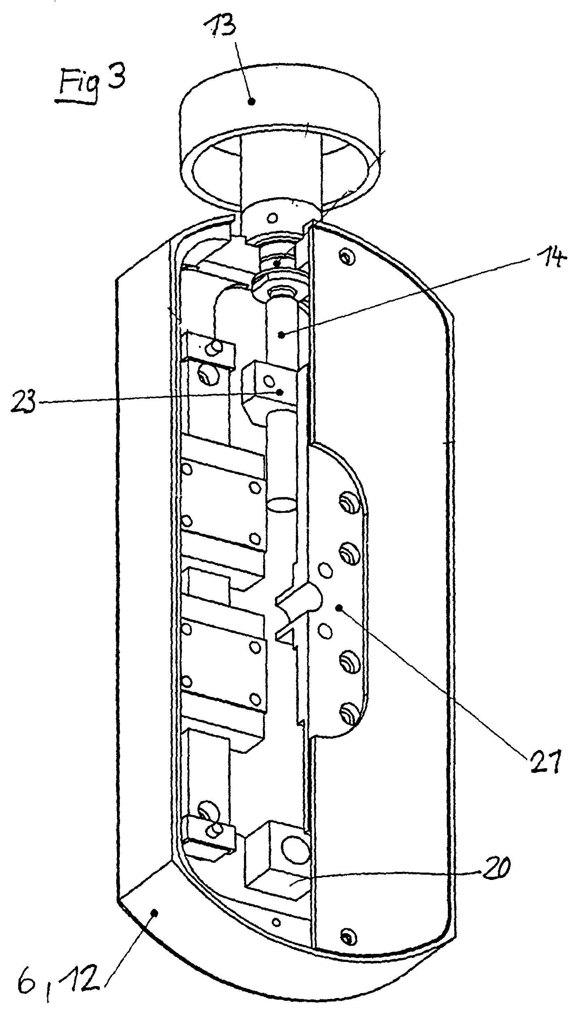

Die Schlittenführung 12 trägt andererseits eine Welle 3b, die einen Träger 1 und einen Arbeitshebel 2 durchsetzt. Der Träger 1 ist weiters über eine Parallelschlittenführung 8a und 8b mit der Aufhängung 7 verbunden. Diese erlaubt einerseits eine kreisbogenförmige Bewegung des Trägers 1, andererseits lässt sie eine y-y Verstellung mittels Schlittenführung 12 zu. Letztere verfügt über einen Verstellknopf 13, eine Verstellspindel 14 und ein Gewindestück 23, das die Aufnahme 21 antreibt. (Fig. 3)

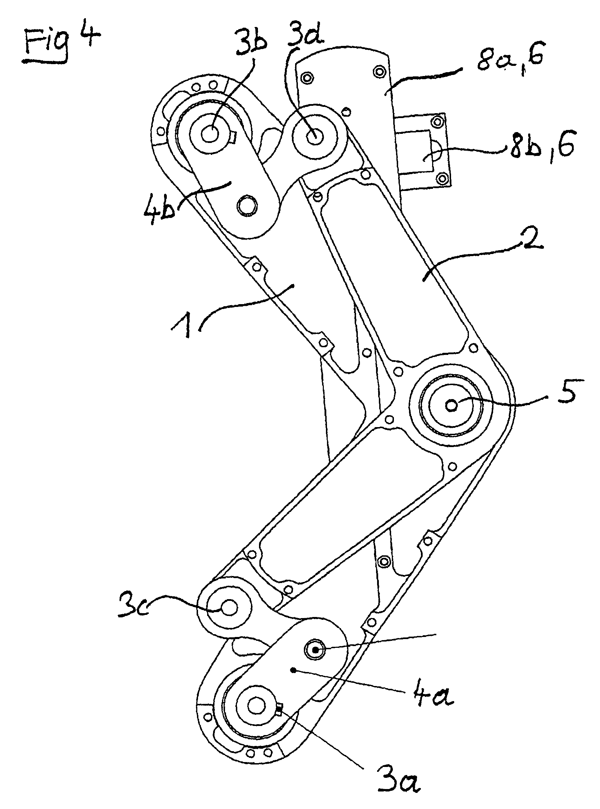

Fig.4 zeigt das Zusammenwirken von Träger 1 und Arbeitshebel 2, der an einer Lagerachse 5 zentral an letzterem gelagert ist. Zwei beidseitig am Arbeitshebel 2 und an den Wellen 3a und 3b gehaltene Kniehebel 4a und 4b übertragen Drehmomente an den Wellen 3a und 3b. Die untere Welle 3a verfügt über ein Anschlussstück 24, das ein Mikroskopinterface 9 aufnimmt. (Fig. 5)

Das Mikroskopinterface 9 trägt eine Neigungsachse 10, die über eine Bremse 11 bremsbar ist. Die Bremse 11 greift dabei über ein Getriebezahnrad 17 auf ein Zahnradsegment 15, das mit der Neigungsachse 10 starr verbunden ist. Das Zahnradsegment 15 ist durch einen Zapfen 16 bewegungsbegrenzt.

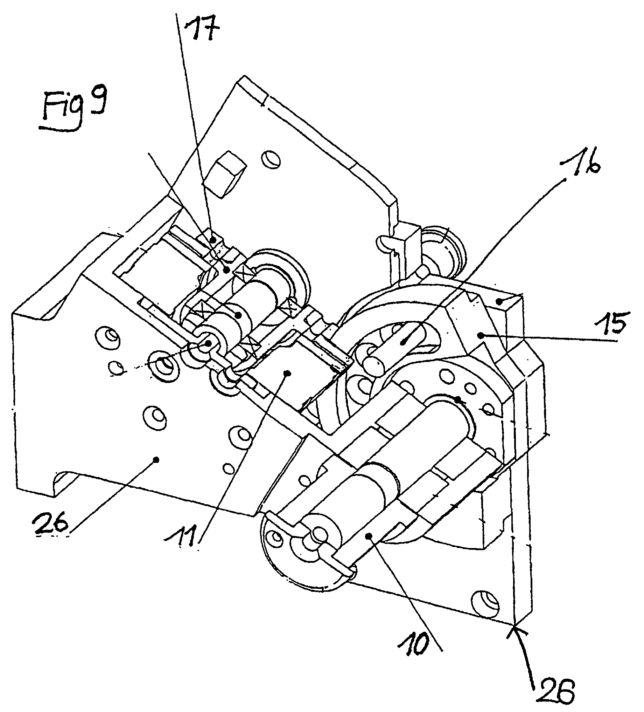

Dadurch wird die Neigebewegung am Operationsmikroskop begrenzt. (Fig. 9)

Weitere Informationen ergeben sich aus der Bezugszeichenliste und den Patentansprüchen.

### Bezugszeichenliste

- 1: Träger
- 2: Arbeitshebel
- 3: Welle a-d
- 4: Kniehebelpaar a,b
- 5: Lagerachse
- 6: Parallelführung
- 7: Aufhängung
- 8: Schlitten a,b
- 9: Mikroskopinterface
- 10: Neigeachse
- 11: Bremse
- 12: Schlittenführung
- 13: Verstellknopf
- 14: Verstellspindel
- 15: Zahnradsegment
- 16: Zapfen
- 17: Zahnrad
- 18: Abdeckung
- 19: Adapter
- 20: Aufnahme für Welle 3b
- 21: Aufnahme für Achse 22
- 22: Achse
- 23: Gewindestück
- 24: Anschlussstück
- 25: Stativinterface
- 26: Mikroskopaufnahme

## Patentansprüche

1. Schwenkträger, geeignet zur Verwendung für ein Operationsmikroskop an einem Stativ mit vertikalen und horizontalen Trägern mit einer Aufhängung, **dadurch gekennzeichnet, dass** er zwei etwa gleichschenkelige zweiarmige Winkelhebel (1,2) umfasst, von denen einer als Trägerhebel (1) den anderen als Arbeitshebel (2) etwa zentral und symmetrisch zu seiner Erstreckung drehbar lagert, so dass auftretende Schwenkmomente vom einen Arbeitshebelende zum anderen 1:1 übertragen werden, wobei die zentrumsfernen Enden der beiden Winkelhebel (1,2) je eine Welle (3) lagern und die beiden jeweils benachbarten Wellen (3a, 3c und 3b, 3d) über je ein identisches Kniehebelpaar (4) miteinander verbunden sind, so dass ein Schwenken des Arbeitshebels (2) zu einem identischen Drehen der beiden Wellen (3a und 3b) führt, die die zentrumsfernen Enden des Trägers (1) durchsetzen.

2. Schwenkträger nach Anspruch 1, **dadurch gekennzeichnet, dass** der Träger (1) durch eine Parallelführung (6) gegenüber Aufhängung (7) abgestützt ist.

3. Schwenkträger nach Anspruch 2, **dadurch gekennzeichnet, dass** die Parallelführung (6) zwei zueinander rechtwinkelig orientierte Gleitschlitten (8) umfasst, die eine kreisbogenförmige Bewegung des Trägers in einer vertikalen Ebene zulassen.

4. Schwenkträger nach einem der vorhergehenden Ansprüche, **dadurch gekennzeichnet, dass** die untere Welle (3a) im Träger (1) ein Mikroskopinterface (9) trägt, welches eine Neigeachse (10) und vorzugsweise eine Bremse (11) dafür umfasst.

5. Schwenkträger nach Anspruch 4, **dadurch gekennzeichnet, dass** die Neigeachse (10) mit einem Zahnradsegment (15) verbunden ist, dessen Neigebewegung durch einen Anschlagzapfen (16) begrenzt ist, und/oder dass die Bremse (11) über ein Zahnrad (17) an das Zahnradsegment (15) angreift.

6. Schwenkträger nach einem der vorhergehenden Ansprüche, **dadurch gekennzeichnet, dass** die obere Welle (3b) im Träger (1) in einer Schlittenführung (12) gehalten ist, die eine y-y-Lageverstellung dieser Welle (3b) und damit des Trägers (1) im Raum erlaubt.

7. Schwenkträger nach Anspruch 5, **dadurch gekennzeichnet, dass** die Schlittenführung (12) einen Verstellknopf (13) mit Gewindespindel (14) und/oder eine automatische Balanciereinrichtung umfasst, die den Schwerpunkt des Schwenkträgers bzw. Mikroskops selbsttätig unter den Aufhängepunkt am Stativ schiebt.

8. Schwenkträger nach einem der vorhergehenden Ansprüche, **dadurch gekennzeichnet, dass** die beweglichen Teile durch wenigstens eine Abdeckungen (18) abgedeckt sind.

9. Schwenkträger nach Anspruch 8, **dadurch gekennzeichnet, dass** die Abdeckung (18) einen Integralschaumstoffbelag aufweist.

## Claims

1. Swivel arm, suitable for use for a surgical operations microscope, at a stand with vertical and horizontal beams with a suspension, **characterised in that** it comprises two double-arm angle levers (1, 2) of approximately equal limb length, of which one, as support lever (1), rotatably mounts the other, as working lever (2), approximately centrally and symmetrically with respect to the length direction thereof so that pivot moments which occur are transferred from one working lever end to the other in a ratio of 1:1, wherein the ends, which are remote from the centre, of the two angle levers (1, 2) each mount a respective shaft (3) and the two respectively adjacent shafts (3a, 3c and 3b, 3d) are interconnected by way of a respective identical toggle lever pair (4) so that a pivotation of the working lever (2) leads to an identical rotation of the two shafts (3a and 3b) which pass through the ends, which are remote from centre, of the beam (1).

2. Swivel arm according to claim 1, **characterised in that** the beam (1) is supported by a parallel guide (6) relative to the suspension (7).

3. Swivel arm according to claim 2, **characterised in that** the parallel guide (6) comprises two slide carriages (8) which are oriented at a right angle relative to one another and which allow a circularly arcuate movement of the arm in a vertical plane.

4. Swivel arm according to one of the preceding claims, **characterised in that** the lower shaft (3a) in the beam (1) carries a microscope interface (9) which has an tilt axle (10) and, preferably, a brake (11) therefor.

5. Swivel arm according to claim 4, **characterised in that** the tilt axle (10) is connected with a segment gear (15), the tilt motion of which is limited by an abutment pin (16), and/or that the brake (11) engages the segment gear (15) by way of a gearwheel (10).

6. Swivel arm according to one of the preceding claims, **characterised in that** the upper shaft (3b) in the beam (1) is mounted in a carriage guide (12) which allows a y-y positional adjustment of this shaft (3b) and thus of the beam (1).

7. Swivel arm according to claim 5, **characterised in that** the carriage guide (12) comprises an adjusting head (13) with a threaded spindle (14) and/or an automatic balancing device which automatically displaces the centre of gravity of the swivel arm or microscope under the suspension point at the stand.

8. Swivel arm according to one of the preceding claims, **characterised in that** the movable parts are covered by at least one cover (18).

9. Swivel arm according to claim 8, **characterised in that** the cover (8) has an integral foamed material coating.

## Revendications

1. Support pivotant, apte à être utilisé pour un microscope d'opération à un pied, avec des supports verticaux et horizontaux avec une suspension, **caractérisé en ce qu'**il comprend deux leviers coudés (1, 2) à deux bras, à branches égales, dont un, en tant que levier de support (1), loge d'une manière rotative l'autre en tant que levier de travail (2) à peu près d'une manière centrale et symétrique à son extension de telle sorte que des couples de pivotement produits sont transmis 1:1 d'une extrémité de levier de travail à l'autre, où les extrémités éloignées du centre des deux leviers coudés (1, 2) logent chacune un arbre (3), et les deux arbres respectivement avoisinants (3a, 3c et 3b, 3d) sont reliés l'un à l'autre par respectivement une paire de leviers coudés identiques (4) de telle sorte qu'un pivotement du levier de travail (2) entraîne une rotation identique des deux arbres (3a et 3b) qui traversent les extrémités éloignées du centre du support (1).

2. Support pivotant selon la revendication 1, **caractérisé en ce que** le support (1) est supporté par un guidage parallèle (6) relativement à la suspension (7).

3. Support pivotant selon la revendication 2, **caractérisé en ce que** le guidage parallèle comprend deux chariots coulissants (8) orientés à angle droit l'un relativement à l'autre qui permettent un déplacement en forme d'arc de cercle du support dans un plan vertical.

4. Support pivotant selon l'une des revendications précédentes, **caractérisé en ce que** l'arbre inférieur (3a) dans le support (1) supporte une interface de microscope (9) qui comprend un axe d'inclinaison (10) et de préférence un frein (11) pour celui-ci.

5. Support pivotant selon la revendication 4, **caractérisé en ce que** l'axe d'inclinaison (10) est relié à un segment de roue dentée (15) dont le mouvement d'inclinaison est limité par un ergot de butée (16) et/ou que le frein (11) s'applique par une roue dentée (17) au segment de roue dentée (15).

6. Support pivotant selon l'une des revendications précédentes, caractérisé en ce gué l'arbre supérieur (3b) est retenu dans le support (1) dans un guidage de chariot (12) qui permet un déplacement de position y-y de cet arbre (3b) et donc du support (1) dans la pièce.

7. Support pivotant selon la revendication 5, **caractérisé en ce que** le guidage de chariot (12) comprend un bouton de réglage (13) avec une tige filetée (14) et/ou une installation d'équilibrage automatique qui pousse le centre de gravité du support pivotant respectivement du microscope automatiquement sous le point de suspension au pied.

8. Support pivotant selon l'une des revendications précédentes, **caractérisé en ce que** les parties mobiles sont recouvertes par au moins un recouvrement (18).

9. Support pivotant selon la revendication 8, **caractérisé en ce que** le recouvrement (18) présente un revêtement en mousse intégral.
